# EUROPEAN PATENT APPLICATION

(11) **EP 4 834 228 A1**
(43) Date of publication of application: **04.11.2026**
(21) Application number: 24927165.1
(22) Date of filing: 27.09.2024
(51) Int. Cl.: A61B 5/346

(54) **PROGRAM, INFORMATION PROCESSING METHOD, AND INFORMATION PROCESSING SYSTEM**

(30) Priority: 01.03.2024 JP 2024031725
(71) Applicant: The University of Tokyo, Bunkyo-ku, Tokyo 113-8654 (JP); Simplex Quantum Inc., Tokyo 150-0031 (JP)
(72) Inventor: FUJIU, Katsuhito, Tokyo 113-8654 (JP); HASUMI, Eriko, Tokyo 113-8654 (JP); SAITO, Ryu, Tokyo 150-0031 (JP); SHIRATSUCHI, Minoru, Tokyo 150-0031 (JP); CHEN, Ying, Tokyo 150-0031 (JP); IKURUMI, Edo, Tokyo 150-0031 (JP)
(74) Representative: Bandpay & Greuter
(86) International application number: PCT/JP2024/034781
(87) International publication number: WO 2025/182126

(57) **Abstract**

To provide a program, an information processing method, an information processing system, and the like, capable of detecting HFpEF at an early stage.

[SOLUTION] According to an aspect of the present invention, there is provided a program configured to cause a computer to execute an obtaining step, a calculation step, and an output step, in which, in the obtaining step, first electrocardiogram data on a first subject is obtained, in the calculation step, based on the first electrocardiogram data and reference information, an index corresponding to HFpEF that is one of heart failure phenotypes classified by a left ventricular ejection fraction, and an index corresponding to an expected value of the left ventricular ejection fraction are calculated, the reference information is information indicating a relationship between second electrocardiogram data on a second subject and the index, in the output step, the calculated indexes are output, and the index corresponding to the HFpEF is at least one of a probability of developing the HFpEF, an expected value calculated from the probability, and information obtained by scaling the expected value to a predetermined range.

## Description

### TECHNICAL FIELD

The present invention relates to a program, an information processing method, and an information processing system.

### BACKGROUND ART

Patent Literature 1 discloses an assay for evaluating heart failure.

This assay will be described. An immunoassay method for detecting and/or monitoring cardiovascular disease in a patient and/or for assessing the likelihood or severity of cardiovascular disease in the patient includes contacting a biological fluid sample from the patient with a monoclonal antibody that specifically binds to a C-terminal epitope of the C5 domain of the α3 chain of type VI collagen, and/or contacting the biological fluid sample from the patient with a monoclonal antibody that specifically binds to a C-terminal neo-epitope of the N-terminal propeptide of type III collagen.

### PRIOR ART LITERATURE

### PATENT LITERATURE

Patent Literature 1: Japanese Patent Laid-Open No. 2022-535513

### SUMMARY OF INVENTION

### TECHNICAL PROBLEM

Heart failure is classified into three phenotypes based on left ventricular ejection fraction. Among these classified phenotypes, HFpEF is heart failure with preserved left ventricular ejection fraction, and thus has been difficult to detect at an early stage.

In view of the above circumstances, the present invention aims to provide a program, an information processing method, an information processing system, and the like, capable of detecting HFpEF at an early stage.

### SOLUTION TO PROBLEM

According to an aspect of the present invention, there is provided a program configured to cause a computer to execute an obtaining step, a calculation step, and an output step, in which, in the obtaining step, first electrocardiogram data on a first subject is obtained, in the calculation step, based on the first electrocardiogram data and reference information, an index corresponding to HFpEF that is one of heart failure phenotypes classified by a left ventricular ejection fraction, and an index corresponding to an expected value of the left ventricular ejection fraction are calculated, the reference information is information indicating a relationship between second electrocardiogram data on a second subject and the index, in the output step, the calculated indexes are output, and the index corresponding to the HFpEF is at least one of a probability of developing the HFpEF, an expected value calculated from the probability, and information obtained by scaling the expected value to a predetermined range.

The above aspect can provide an improvement in the control functions of a computer in the technical field related to heart failure, in that early detection of HFpEF, which is particularly difficult to detect at an early stage, is enabled. In other words, according to this aspect, the functions of a computer can be improved so as to achieve at least one of the following (1) to (4). (1) The processing of the computer can be accelerated. (2) The power consumption of the computer can be reduced. (3) The communication speed of the computer can be increased. (4) Resources saved on the computer can be used for other core functions.

According to the aspect as described above, HFpEF can be detected at an early stage.

### BRIEF DESCRIPTION OF DRAWINGS

FIG. 1 is a configuration diagram illustrating an information processing system 100.
FIG. 2 is a block diagram illustrating the hardware configuration of an information processing apparatus 200.
FIG. 3 is a block diagram illustrating the hardware configuration of a terminal 300.
FIG. 4 is a block diagram illustrating functions realized by the information processing apparatus 200 (a control unit 210).
FIG. 5 is an activity diagram illustrating the flow of an information processing method to be executed by the information processing apparatus 200.
FIG. 6 is a diagram illustrating the content displayed on a display unit 330 of the terminal 300.

### DESCRIPTION OF EMBODIMENT

Hereinafter, an embodiment of the present invention will be described with reference to the drawings. The various features illustrated in the embodiment described below can be combined with one another.

A program for realizing software described in an embodiment may be provided as a non-transitory computer-readable medium, may be provided to be downloadable from an external server, or may be provided in a manner where the program is launched on an external computer to realize its functions on a client terminal (so-called cloud computing).

In addition, in various types of information processing according to an embodiment, an input and an output corresponding to the input can be realized. Here, as long as an output is obtained as a result of an input, the form of the information referred to in the information processing as described above (hereinafter referred to as "the reference information") is not limited. The reference information may be, for example, rule-based information such as a database, a lookup table, or a predetermined function (including a decision formula, such as a regression equation, constructed using a statistical method), a trained model that has learned the correlation between inputs and outputs in advance, or a large language model capable of outputting a desired result in response to an input of a prompt.

In addition, in one embodiment, "unit" may encompass, for example, a combination of hardware resources implemented by a circuit in a broad sense and software information processing that can be specifically realized by these hardware resources. In addition, while various items of information are handled in an embodiment, such information may be represented, for example, by physical values of signal values indicating voltage/current, by high and low of a signal value as a collection of binary bits consisting of 0s and 1s, or by quantum superposition (so-called qubits), and communication and computation may be executed on a circuit in a broad sense.

Furthermore, a circuit in a broad sense is a circuit realized by appropriately combining at least a circuit, circuitry, a processor, memory, and the like. In addition, the processor may be a general-purpose processor or a dedicated circuit. In other words, it includes an application-specific integrated circuit (ASICs), a programmable logic device (such as a simple programmable logic devices (SPLD), a complex programmable logic device (CPLD), and a field-programmable gate array (FPGA)), and the like.

### 1. Hardware Configuration

In Section 1, the hardware configuration of the present embodiment will be described.

### 1-1. Information Processing System 100

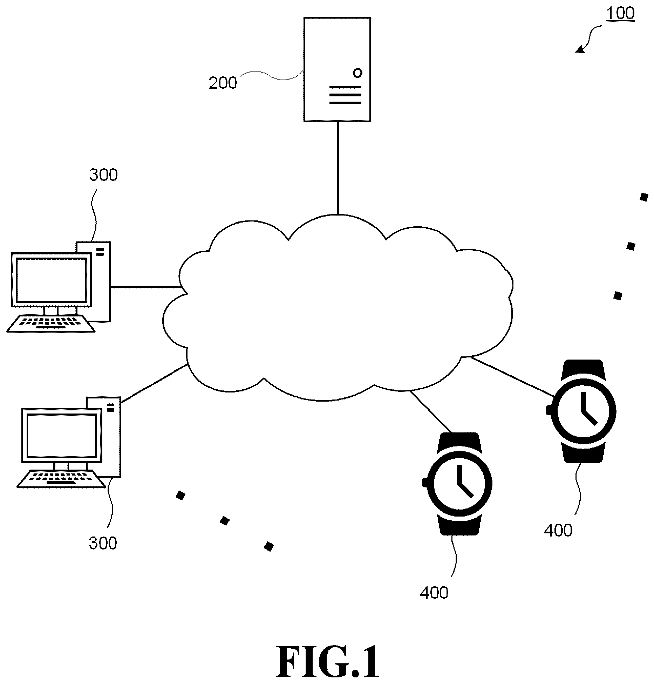

FIG. 1 is a configuration diagram illustrating an information processing system 100. The information processing system 100 includes an information processing apparatus 200, terminals 300, and electrocardiographs 400, and these are connected via a network. These components will be further described. Here, a system exemplified by the information processing system 100 consists of one or more apparatuses or components. Accordingly, for example, even the information processing apparatus 200 alone can constitute a system exemplified by the information processing system 100.

### 1-2. Information Processing Apparatus 200

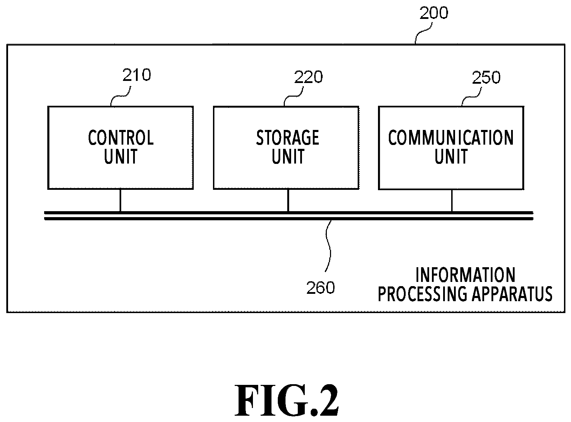

FIG. 2 is a block diagram illustrating the hardware configuration of the information processing apparatus 200. The information processing apparatus 200 includes a control unit 210, a storage unit 220, and a communication unit 250, and these components are electrically connected within the information processing apparatus 200 via a communication bus 260. Each component will be further described.

The control unit 210 performs processing and control of the overall operations related to the information processing apparatus 200. The control unit 210 is, for example, a central processing unit (CPU) that is not illustrated. The control unit 210 realizes various functions related to the information processing apparatus 200 by reading a predetermined program stored in the storage unit 220. In other words, since the information processing performed by software stored in the storage unit 220 is concretely realized by the control unit 210, which is an example of hardware, it may be executed as each functional unit included in the control unit 210. These will be further described in Section 2. It should be noted that the control unit 210 is not limited to being a single unit, and may be implemented such that a plurality of control units 210 is implemented, each corresponding to a specific function. In addition, it may be a combination of these.

The storage unit 220 stores various items of information necessary for the information processing of the information processing apparatus 200. This may be implemented, for example, as a storage device such as a solid-state drive (SSD) that stores various programs and the like related to the information processing apparatus 200 to be executed by the control unit 210, or as a memory such as a random access memory (RAM) that stores information (arguments, arrays, and the like) temporarily required for program calculations. In addition, it may be a combination of these.

The communication unit 250 is preferably wired communication means such as USB, IEEE1394, Thunderbolt (registered trademark), or wired LAN network communication, but may also include wireless LAN network communication, mobile communication such as 5G / LTE / 3G, Bluetooth (registered trademark) communication, and the like, as needed. In other words, it is more preferable to implement it as a combination of a plurality of these types of communication means. In other words, the information processing apparatus 200 communicates various types of information with the terminals 300 via the communication unit 250 and a network.

### 1-3. Terminal 300

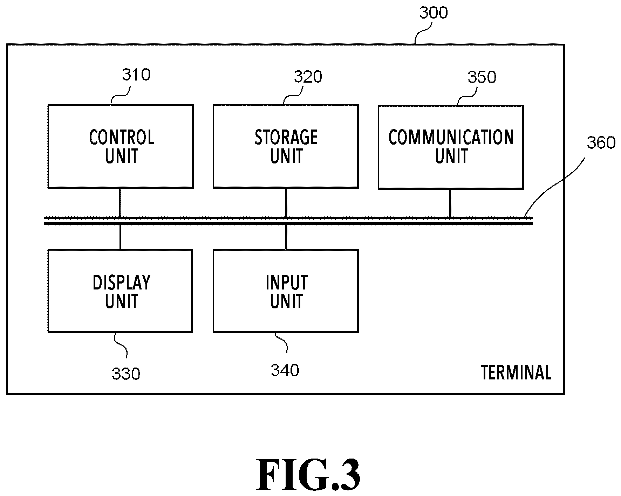

FIG. 3 is a block diagram illustrating the hardware configuration of the terminal 300. The terminal 300 includes a control unit 310, a storage unit 320, a display unit 330, an input unit 340, and a communication unit 350, and these components are electrically connected within the terminal 300 via a communication bus 360. Descriptions of the control unit 310, the storage unit 320, and the communication unit 350 are omitted, since they are substantially the same as the descriptions of the control unit 210, the storage unit 220, and the communication unit 250 in the information processing apparatus 200. It should be noted that the terminal 300 may be, for example, a desktop computer, a laptop computer, a smartphone, a tablet terminal, or the like.

The display unit 330 may be included in a housing of the terminal 300 or may be externally attached to the terminal 300. The display unit 330 displays a screen of a graphical user interface (GUI) that the user can operate. This is preferably implemented by selectively use a display device such as a CRT display, a liquid crystal display, an organic EL display, and a plasma display, depending on the type of the terminal 300. Hereinafter, the display unit 330 will be described as being included in the housing of the terminal 300.

The input unit 340 may be included in the housing of the terminal 300 or may be externally attached to the terminal 300. For example, the input unit 340 may be implemented as a touch panel integrated with the display unit 330. With a touch panel, a user can input a tapping operation, a swiping operation, and the like. Of course, instead of a touch panel, a switch button, a mouse, a QWERT keyboard, and the like may be used. In other words, the input unit 340 accepts operation inputs made by the user. The inputs are transferred to the control unit 310 via the communication bus 360 as command signals. In addition, the control unit 310 can execute predetermined control or calculations as needed.

### 1.4 Electrocardiograph 400

The electrocardiograph 400 is configured to obtain, as electrocardiogram data, data related to temporal fluctuations in the action potentials of cardiac muscle cells associated with heartbeats. The electrocardiograph 400 may be, for example, a 12-lead electrocardiograph, a wearable terminal equipped with electrocardiograph functions, or the like, and a vector electrocardiograph, a long-term recording electrocardiograph, a body surface potential meter, an automatic electrocardiograph, or the like may be appropriately selected depending on the intended use.

The electrocardiograph 400 is connected to the communication unit 250 of the information processing apparatus 200 via a network, and is configured to be able to transfer the obtained electrocardiogram data to the information processing apparatus 200. It should be noted that the electrocardiograph 400 may not include a communication unit. In this case, the obtained electrocardiogram data may be recorded on a recording medium such as a memory card, transferred from the recording medium to the terminal 300, and transferred from the terminal 300 to the information processing apparatus 200.

### 2. Functional Configuration

In Section 2, the functional configuration of the present embodiment will be described. As previously described, since the information processing by the software stored in the storage unit 220 is concretely realized by the control unit 210, which is an example of hardware, the information processing can be executed as various functional units included in the control unit 210.

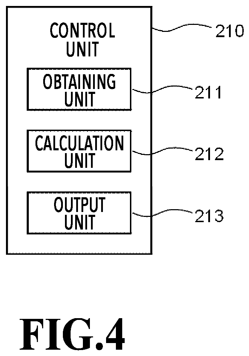

FIG. 4 is a block diagram illustrating functions realized by the information processing apparatus 200 (the control unit 210). As described above, the information processing apparatus 200 (the information processing system) includes the control unit 210. Specifically, the information processing apparatus 200 (the control unit 210) is configured to execute each step of the program in the present embodiment. The information processing apparatus 200 (the control unit 210) includes an obtaining unit 211, a calculation unit 212, and an output unit 213 corresponding to steps of the program in the present embodiment. Here, the program in the present embodiment is configured to cause a computer, such as the information processing apparatus 200, to execute an obtaining step, a calculation step, and an output step.

The obtaining unit 211 is configured to obtain various types of information. The obtaining unit 211 is configured to execute the obtaining step. For example, the obtaining unit 211 obtains first electrocardiogram data on a first subject from the electrocardiograph 400.

The calculation unit 212 is configured to calculate various types of information. The calculation unit 212 is configured to execute the calculation step. For example, based on the obtained first electrocardiogram data and the reference information, the calculation unit 212 calculates an index corresponding to HFpEF (hereinafter also referred to as "the HFpEF index"), which is one of the heart failure phenotypes classified by left ventricular ejection fraction. The reference information is information indicating the relationship between second electrocardiogram data on a second subject different from the first subject and the index.

Here, the heart failure phenotypes based on the left ventricular ejection fraction are classified into three, i.e., HFpEF, HFrEF, and HFmrEF. HFpEF (heart failure with preserved ejection fraction) is defined as heart failure with a preserved left ventricular ejection fraction, and is defined with a left ventricular ejection fraction of 50% or more. HFrEF (heart failure with reduced ejection fraction) is defined as heart failure with a reduced left ventricular ejection fraction, and is defined with a left ventricular ejection fraction of less than 40%. HFmrEF (heart failure with mid-range ejection fraction) is defined as heart failure with a mildly reduced left ventricular ejection fraction, and is defined with a left ventricular ejection fraction of 40% or more and less than 50%.

The output unit 213 is configured to output various types of information. The output unit 213 is configured to execute the output step. For example, the output unit 213 outputs the calculated index to the terminal 300.

### 3. Information Processing Method

In Section 3, a description will be given of the flow of an information processing method to be executed by the aforementioned information processing apparatus 200. This information processing method includes each step of the program in the present embodiment. This information processing method includes an obtaining step, a calculation step, and an output step. In the obtaining step, the first electrocardiogram data on the first subject is obtained. In the calculation step, an index corresponding to HFpEF, which is one of the heart failure phenotypes classified by the left ventricular ejection fraction, is calculated based on the obtained first electrocardiogram data and the reference information. The reference information is information indicating the relationship between the second electrocardiogram data on the second subject and the index. In the output step, the calculated index is output.

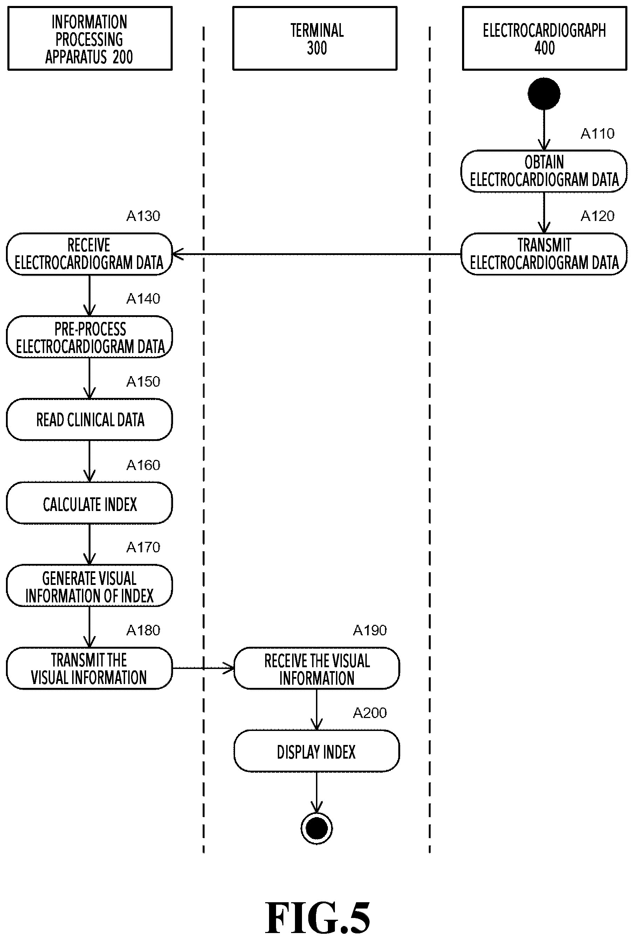

FIG. 5 is an activity diagram illustrating the flow of the information processing method to be executed by the information processing apparatus 200. Hereinafter, a description will be given along each activity in the activity diagram.

The electrocardiograph 400 obtains the first electrocardiogram data on the first subject, which is an arbitrary subject (Activity A110).

The first electrocardiogram data may consist of 1 to 50 beats. Specifically, the first electrocardiogram data consists of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50 beats, and may fall within a range between any two of the numerical values exemplified here. Preferably, the first electrocardiogram data consists of 1 to 2 beats. In this manner, the information processing apparatus 200 can calculate and output an HFpEF index even with a small amount of data. Accordingly, the time required to obtain the first electrocardiogram data can be reduced.

The first electrocardiogram data may be composed of a waveform over a duration of 5 to 300 seconds. Specifically, it is composed of a waveform over a duration of, for example, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, or 300 seconds, and may fall within a range between any two of the numerical values exemplified here. Preferably, the first electrocardiogram data is composed of a waveform over a duration of 5 to 10 seconds. In this manner, the information processing apparatus 200 can calculate and output an HFpEF index even with a small amount of data. Accordingly, the time required to obtain the first electrocardiogram data can be reduced.

The first electrocardiogram data may be data obtained from an electrode corresponding to any one of 12 leads. Preferably, the one lead is Lead I obtained from the right arm and left arm of the first subject. Here, "the right arm" refers to the area extending from the right hand to the base of the right arm. Similarly, "the left arm" refers to the area extending from the left hand to the base of the left arm. Accordingly, the placement position of the electrode for obtaining Lead I may be any position from the hand to the base of the arm. According to the aspect as described above, it is possible to use Lead I that can be easily obtained. Accordingly, it is possible to suppress the increase in the burden on the first subject associated with the obtaining of the first electrocardiogram data.

Subsequently, the electrocardiograph 400 transmits the obtained first electrocardiogram data to the information processing apparatus 200 via the network (Activity A120).

Next, the control unit 210 in the information processing apparatus 200 receives the first electrocardiogram data transmitted from the electrocardiograph 400 (Activity A130). In terms of steps, in the obtaining step, the first electrocardiogram data on the first subject is obtained. In Activity A130, for example, the following two stages of information processing are executed. (1) The communication unit 250 receives the first electrocardiogram data transmitted from the electrocardiograph 400. (2) The control unit 210 causes the storage unit 220 to store the received first electrocardiogram data.

Subsequently, the control unit 210 in the information processing apparatus 200 pre-processes the first electrocardiogram data (Activity A140). Here, the pre-processing includes, for example, detrending, motion artifact removal, noise removal, segmentation of waveform data on a heartbeat-by-heartbeat basis, normalization, and the like, and is executed by appropriately combining these. Preferably, the pre-processing is executed in the order of detrending, motion artifact removal, noise removal, segmentation of waveform data on a heartbeat-by-heartbeat basis, and normalization. In Activity A140, for example, the following three stages of information processing are executed. (1) The control unit 210 reads the first electrocardiogram data and a predetermined parameter from the storage unit 220. (2) The control unit 210 applies the predetermined parameter to the raw data of the first electrocardiogram data, and pre-processes the first electrocardiogram data. (3) The control unit 210 causes the storage unit 220 to store the pre-processed first electrocardiogram data. According to Activity A140, the accuracy of the HFpEF index can be improved by removing noise contained in the first electrocardiogram data.

Next, the control unit 210 in the information processing apparatus 200 reads first clinical data on the first subject (Activity A150). Here, the first clinical data may be data including at least one of age, gender, BMI (Body Mass Index), PWTT (Pulse Wave Transit Time), blood pressure, heart rate, SDNN (Standard Deviation of the NN interval), CVRR (Coefficient of Variation of RR interval), atrial fibrillation, and HRV (Heart Rate Variability). The same applies to second clinical data, which will be described later. In Activity A150, for example, the following information processing is executed. The control unit 210 reads the first clinical data from the storage unit 220. According to Activity A150, the accuracy of the HFpEF index can be improved by using the first clinical data in addition to the first electrocardiogram data.

Subsequently, the control unit 210 in the information processing apparatus 200 calculates the HFpEF index based on the first electrocardiogram data on the first subject, the first clinical data on the first subject, and the reference information (Activity A160). In terms of steps, in the calculation step, an index is calculated that corresponds to HFpEF, which is one of the heart failure phenotypes classified by the left ventricular ejection fraction, based on the first electrocardiogram data, the first clinical data on the first subject, and the reference information. The HFpEF index may be any information useful for detecting HFpEF, such as the probability of developing HFpEF, an expected value calculated from the probability, or information derived by scaling the expected value to a predetermined range. In Activity A160, for example, the following three stages of information processing are executed. (1) The control unit 210 further reads the pre-processed first electrocardiogram data and the reference information from the storage unit 220. (2) The control unit 210 inputs the pre-processed first electrocardiogram data and the first clinical data to the reference information, and calculates the HFpEF index. (3) The control unit 210 causes the storage unit 220 to store the calculated HFpEF index.

Here, the reference information is information indicating the relationship among the second electrocardiogram data on the second subject, the second clinical data on the second subject, and the HFpEF index. Preferably, the reference information is a trained model that has learned the relationship among the second electrocardiogram data, the second clinical data, and the HFpEF index. By using the trained model as described above, the accuracy of HFpEF detection can be further improved.

Here, the second subject includes a subject who has developed HFpEF but has no subjective symptoms of heart failure. In other words, the trained model in the present embodiment has been trained not only on electrocardiogram data from subjects experiencing subjective symptoms of heart failure due to the onset of HFpEF (subjects classified as "Class II" to "Class IV" of NYHA) but also on electrocardiogram data from subjects who previously developed HFpEF and experienced subjective symptoms of heart failure but currently do not exhibit subjective symptoms of heart failure (subjects currently classified as "Class I" of NYHA). In other words, the trained model in the present embodiment has been trained on diverse electrocardiogram data related to HFpEF. Accordingly, even in cases of early-stage HFpEF that are difficult to diagnose via ultrasound examination, the HFpEF index is output, and thus it is possible to present the HFpEF index as useful information during HFpEF screening. According to the aspect as described above, HFpEF can be more accurately detected even in patients who do not exhibit subjective symptoms, such as patients classified as "Class I" of NYHA or patients in the early stage of developing a first episode of HFpEF.

Subsequently, the control unit 210 in the information processing apparatus 200 generates visual information related to the HFpEF index (hereinafter also simply referred to as "the visual information") as information viewable on the terminal 300 (Activity A170). In activity A170, for example, the following three stages of information processing are executed. (1) The control unit 210 reads the HFpEF index from the storage unit 220. (2) The control unit 210 executes generation processing and generates the visual information. (3) The control unit 210 causes the storage unit 220 to store the visual information.

Next, the control unit 210 in the information processing apparatus 200 transmits the visual information related to the HFpEF index to the terminal 300 (Activity A180). In terms of steps, in the output step, the calculated index is output. In Activity A180, for example, the following two stages of information processing are executed. (1) The control unit 210 reads the visual information from the storage unit 220. (2) The control unit 210 transmits the visual information to the terminal 300 via the communication unit 250.

Subsequently, the control unit 310 in the terminal 300 receives the visual information transmitted from the information processing apparatus 200 (Activity A190). In Activity A190, for example, the following two stages of information processing are executed. (1) The communication unit 350 receives the visual information transmitted from the information processing apparatus 200. (2) The control unit 310 causes the storage unit 320 to store the received visual information.

Subsequently, the control unit 310 in the terminal 300 causes the display unit 330 to display the HFpEF index (Activity A200). In Activity A200, for example, the following two stages of information processing are executed. (1) The control unit 310 reads the received visual information from the storage unit 320. (2) The control unit 310 inputs the visual information to the display unit 330 via the communication bus 360. (3) The display unit 330 displays the HFpEF index.

### 4. Display Example

In Section 4, display examples of the HFpEF index will be described.

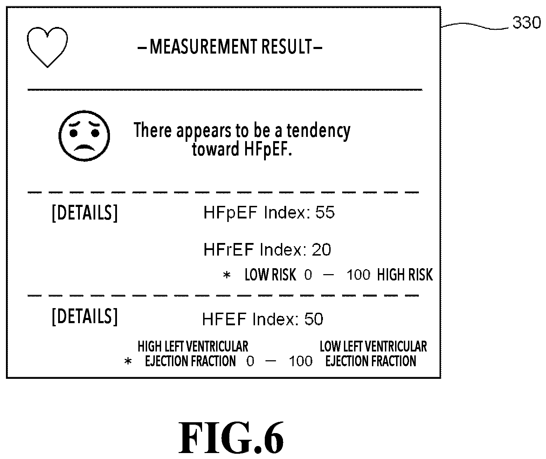

FIG. 6 is a diagram illustrating the content displayed on the display unit 330 of the terminal 300. The display unit 330 displays an HFrEF index in addition to the HFpEF index, and further displays an HFEF index. Here, the HFEF index represents an index corresponding to the expected value of the left ventricular ejection fraction, as a concept that encompasses the heart failure phenotypes. In FIG. 6, the HFEF index is calculated by using the respective probabilities of HFpEF and HFrEF, and may be calculated by further using the probability of HFmrEF. In other words, in FIG. 6, in Activity A160, in the calculation step, an index corresponding to at least one of HFrEF and HFmrEF, which are phenotypes other than HFpEF, and an index corresponding to the expected value of the left ventricular ejection fraction are further calculated.

Each index displayed on the display unit 330 is set within a range of 0 to 100. The HFpEF index indicates that the closer it is to 0, the lower the risk of HFpEF, and the closer it is to 100, the higher the risk of HFpEF. Similarly, the HFrEF index indicates that the closer it is to 0, the lower the risk of HFrEF, and the closer it is to 100, the higher the risk of HFrEF. The HFEF index estimates the degree of left ventricular ejection fraction in heart failure, and indicates that the closer it is to 0, the higher the left ventricular ejection fraction, and that the closer it is to 100, the lower the left ventricular ejection fraction. In other words, the HFEF index is an index calculated and displayed when the first subject is a patient who has developed heart failure.

In FIG. 6, since the HFEF index is "50," it is indicated that the degree of left ventricular ejection fraction in heart failure is moderate. In addition, since the HFpEF index is "55" while the HFrEF index is "20", and the HFpEF index is higher, it can be seen that the tendency to develop HFpEF is stronger than the tendency to develop HFrEF. Therefore, the display unit 330 in FIG. 6 displays "There appears to be a tendency toward HFpEF." as the measurement result of the electrocardiogram data. According to the aspect as described above, it is possible to present the risk of each heart failure phenotype.

According to the aspect as described above, it is possible to detect HFpEF, which is particularly difficult to diagnose, at an early stage. In addition, due to the simple configuration, the saved resources can be used for other core functions.

While the embodiment of the present invention has been described above, the present invention is not limited to the embodiment, and can be appropriately modified within a range that does not depart from the technical idea of the invention.

### 5. Variations

In Section 5, variations of the present embodiment will be described. The following variations can be appropriately combined.

An aspect of the present embodiment may be a recording medium. The recording medium is a computer-readable non-transitory recording medium. The program in this embodiment is recorded in the recording medium.

The control unit 210 performs write (storing) processing and read processing regarding various types of data and various types of information on the storage unit 220, but is not limited to this, and the information processing for each activity may be executed using, for example, registers and cache memory in the control unit 210.

The output by the output unit 213 is not limited to transmission processing of the visual information related to the index to the terminal 300, and may be, for example, displaying the visual information on the display device of the information processing apparatus 200.

The range of the index is not limited to 0 to 100, and may be, for example, a range of 0 to 1, or a range of 0 to 10, as long as the range is appropriately set. In addition, the degree of weighting for each index can be appropriately set, and the values may be inverted. For example, the HFEF index closer to 0 may indicate a lower left ventricular ejection fraction during heart failure, while the HFEF index closer to 100 may indicate a higher left ventricular ejection fraction during heart failure.

The reference information is not limited to trained models, and may be a database storing lookup tables and the like, a function representing the correspondence of values determined based on a certain variable, or a mathematical model that mathematically relates a plurality of items of information.

The electrocardiograph 400 may be configured to be able to communicate with the information processing apparatus 200 via the terminal 300. In this case, the electrocardiograph 400 may be connected to the communication unit 350 of the terminal 300 via a network, or may take the form of being directly connected to the terminal 300.

The output unit 213 may be configured to output other items in addition to the HFpEF index. For example, the output unit 213 may further output the blood sampling result of the first subject.

The reference information may be information indicating the relationship between the second electrocardiogram data on the second subject and the HFpEF index, independently of the clinical data on the second subject. In this case, the reference information may be a trained model that has learned the relationship between the second electrocardiogram waveform and the index.

The output unit 213 may display only the HFpEF index as the index of the heart failure phenotype. In other words, as illustrated in Activity A160, only the HFpEF index may be calculated. According to the aspect as described above, the visibility can be improved when it is desired to confirm whether HFpEF has been developed.

The first electrocardiogram data is not limited to data using Lead I, but may also be data using other leads. In addition, the first electrocardiogram data may also be data using a plurality of leads. The more the number of leads used, the more the accuracy of the index can be improved. According to the aspect as described above, electrocardiogram data can be easily obtained, and the number of leads used can be adjusted according to the purpose.

### 6. Others

The embodiment may also be provided in the aspects described below.
(1) A program configured to cause a computer to execute an obtaining step, a calculation step, and an output step, in which, in the obtaining step, first electrocardiogram data on a first subject is obtained, in the calculation step, based on the first electrocardiogram data and reference information, an index corresponding to HFpEF that is one of heart failure phenotypes classified by a left ventricular ejection fraction, and an index corresponding to an expected value of the left ventricular ejection fraction are calculated, the reference information is information indicating a relationship between second electrocardiogram data on a second subject and the index, in the output step, the calculated indexes are output, and the index corresponding to the HFpEF is at least one of a probability of developing the HFpEF, an expected value calculated from the probability, and information obtained by scaling the expected value to a predetermined range.
   According to the aspect as described above, HFpEF can be detected at an early stage. In addition, due to the simple configuration, the saved resources can be used for other core functions.
(2) The program according to (1) described above, in which the reference information is a trained model that has learned a relationship between the second electrocardiogram data and the index.
   According to the aspect as described above, the accuracy of HFpEF detection can be improved.
(3) The program according to (1) or (2) described above, in which the second subject includes a subject who has developed HFpEF but has no subjective symptoms of heart failure.
   According to the aspect as described above, HFpEF can be more accurately detected even in patients who do not exhibit subjective symptoms, such as patients classified as "Class I" of NYHA or patients in the early stage of developing a first episode of HFpEF.
(4) The program according to any one of (1) to (3) described above, in which the first electrocardiogram data is data obtained from an electrode corresponding to any one lead of 12 leads.
   According to the aspect as described above, electrocardiogram data can be easily obtained.
(5) The program according to (4) described above, in which the one lead is Lead I obtained from a right arm and a left arm of the first subject.
   According to the aspect as described above, it is possible to use Lead I that can be easily obtained.
(6) The program according to any one of (1) to (5) described above, in which, in the calculation step, the index is calculated based on the first electrocardiogram data, first clinical data on the first subject, and the reference information, and the reference information is information indicating a relationship among the second electrocardiogram data, second clinical data on the second subject, and the index.
   According to the aspect as described above, the accuracy of HFpEF detection can be further improved.
(7) The program according to (6) described above, in which the reference information is a trained model that has learned the relationship among the second electrocardiogram data, the second clinical data, and the index.
   According to the aspect as described above, the accuracy of HFpEF detection can be further improved.
(8) The program according to any one of (1) to (7), in which, in the calculation step, an index is further calculated that corresponds to at least one of HFrEF and HFmrEF that are the phenotypes other than the HFpEF.
   According to the aspect as described above, it is possible to present the risk of each heart failure phenotype.
(9) An information processing method including each step of the program according to any one of (1) to (8) described above.
   According to the aspect as described above, HFpEF can be detected at an early stage. In addition, due to the simple configuration, the saved resources can be used for other core functions.
(10) An information processing system including a control unit, in which the control unit is configured to execute each step of the program according to any one of (1) to (8) described above.
   According to the aspect as described above, HFpEF can be detected at an early stage. In addition, due to the simple configuration, the saved resources can be used for other core functions.
   Of course, this is not a limitation.

### Reference Signs List

100: information processing system, 200: information processing apparatus, 210: control unit, 211: obtaining unit, 212: calculation unit, 213: output unit, 220: storage unit, 250: communication unit, 260: communication bus, 300: terminal, 310: control unit, 320: storage unit, 330: display unit, 340: input unit, 350: communication unit, 360: communication bus, 400: electrocardiograph

## Claims

1. A program, configured to cause a computer to execute an obtaining step, a calculation step, and an output step, wherein
in the obtaining step, first electrocardiogram data on a first subject is obtained,
in the calculation step, based on the first electrocardiogram data and reference information, an index corresponding to HFpEF that is one of heart failure phenotypes classified by a left ventricular ejection fraction, and an index corresponding to an expected value of the left ventricular ejection fraction are calculated,
the reference information is information indicating a relationship between second electrocardiogram data on a second subject and the indexes,
in the output step, the calculated indexes are output, and
the index corresponding to the HFpEF is at least one of a probability of developing the HFpEF, an expected value calculated from the probability, and information obtained by scaling the expected value to a predetermined range.

2. The program according to claim 1,
wherein the reference information is a trained model that has learned a relationship between the second electrocardiogram data and the indexes.

3. The program according to claim 1 or 2,
wherein the second subject includes a subject who has developed the HFpEF but has no subjective symptoms of heart failure.

4. The program according to any one of claims 1 to 3,
wherein the first electrocardiogram data is data obtained from an electrode corresponding to any one lead of 12 leads.

5. The program according to claim 4,
wherein the one lead is Lead I obtained from a right arm and a left arm of the first subject.

6. The program according to any one of claims 1 to 5,
wherein in the calculation step, the index is calculated based on the first electrocardiogram data, first clinical data on the first subject, and the reference information, and
wherein the reference information is information indicating a relationship among the second electrocardiogram data, second clinical data on the second subject, and the index.

7. The program according to claim 6,
wherein the reference information is a trained model that has learned the relationship among the second electrocardiogram data, the second clinical data, and the index.

8. The program according to any one of claims 1 to 7,
wherein in the calculation step, an index is further calculated that corresponds to at least one of HFrEF and HFmrEF that are the phenotypes other than the HFpEF.

9. An information processing method comprising:
each step of the program according to any one of claims 1 to 8.

10. An information processing system, comprising:
a control unit,
wherein the control unit is configured to execute each step of the program according to any one of claims 1 to 8.
